# EUROPEAN PATENT APPLICATION

(11) **EP 1 726 318 A1**
(43) Date of publication of application: **29.11.2006**
(21) Application number: 05721433.0
(22) Date of filing: 17.03.2005
(51) Int. Cl.: A61L 27/00, A61K 38/00, A61K 38/16, A61K 38/22, A61K 47/48, A61K 48/00, A61P 9/00, A61P 9/10

(54) **MEDICINE/GENE LEACHING TYPE STENT**

(30) Priority: 18.03.2004 JP 2004077581
(71) Applicant: Egashira, Kensuke, Fukuoka-shi Fukuoka 814-0001 (JP)
(72) Inventor: Egashira, Kensuke, Fukuoka-shi Fukuoka 814-0001 (JP)
(74) Representative: HOFFMANN EITLE
(86) International application number: PCT/JP2005/005456
(87) International publication number: WO 2005/089822

(57) **Abstract**

The present invention provides a more safe and highly effective stent having functions such as anti-inflammatory action, antithrombotic action, maintenance of tissue restoration response and maintenance of endothelial regeneration. More speciffically, the drug/gene eluting stent has a layer containing a gene encoding a hybrid polypeptide on the surface. The hybrid polypeptide is preferably a bound of fibronectin-derived collagen binding domain (FNCBD) polypeptide and an anti-inflammatory factor or an angiogenic factor. The uniform fine particle size capsules can directly deliver the gene encoding the hybrid polypeptide to the lesion and have benefits of reducing the given doses, and thus improving safety and efficacy and further maintaining the efficacy for a long period.

## Description

### Technical Field of the Invention

The present invention relates to a drug/gene eluting stent.

### Prior Art

The fundamental pathology of angina pectoris and cardiac infarction is a stenosis and thrombosis of cardiac artery due to arteriosclerosis, and coronary interventions (PCI, balloon dilation and stent dilation) dilating arteriosclerotic stenosis have been popularly used as a useful therapy in the world.

However, a high rate of "restenosis" or relapsed stenosis of the once dilated cardiac artery has become a medical problem. The recurrent cardiac ischemia due to restenosis may highly causes relapsed angina pectoris and cardiac infarction, and not a few cases require re-operation of PCI or coronary artery bypass surgery.

Therefore, a new therapeutic approach inhibiting the restenosis has been expected, however, no established method has been found.

WO-A 02/47739, JP-A 2002-060900, JP-A 2002-289698, Artif. Organs, 2003 Feb.; 27(2): 147-54, and Nat. Biotechnol., 2000 Nov.; 18(11): 1181-4 may be referred.

A drug eluting stent prepared by coating the stent with a restenosis inhibiting agent has recently been paid attention as an improved measure, and application of such a coating stent is expected to provide a restenosis inhibitory factor to the local lesion for a long period. A stent coated with an anticancer and antiproliferation drug sirolimus (rapamycin) was reported in N. Engl. J. Med., June 2002. However, it has some drawbacks such as emergence of dead cases due to subacute stage thrombosis, hypersensitivities (such as pain, eczema and blood pressure fluctuation), persistent chronic inflammation and delayed endothelial regeneration/vascular reparation reaction, and no sufficient improvement has been obtained.

Furthermore, the above mentioned reference also disclosed an invention with similar purpose, but no satisfactory result was also obtained.

### Disclosure of the Invention

The problem to be solved by the present invention is to provide a safer and excellent effective drug/gene eluting stent with functions of anti-inflammatory action, antithrombotic action, maintenance of tissue reparation reaction and/or endothelial regeneration function.

The present invention provides, as a method for solving the problem, a drug/gene eluting stent with a surface layer containing a gene encoding a hybrid polypeptide.

The present invention further provides a method for the treatment of vascular restenosis, acute coronary syndromes or cerebral ischemia, which comprises using the above-mentioned drug/gene eluting stent or a use for the preparation of the above-mentioned drug/gene eluting stent as a therapeutic agent for the vascular restenosis, acute coronary syndromes or cerebral ischemia.

The layer containing the gene encoding a hybrid polypeptide in the present invention is composed of at least one layer, i.e., one layer or a combination of two or more layers and is/are formed on whole or a part of the stent surface.

### Detailed Description of the Invention

The drug/gene eluting stent of the present invention has a layer containing the gene encoding a hybrid polypeptide on the surface.

The hybrid polypeptide is preferably a bound product of a polypeptide of fibronectin-derived collagen binding domain (FNCBD) and an anti-inflammatory factor or an angiogenic factor. The fibronectin-derived collagen binding domain (FNCBD) is a polypeptide disclosed in JP-A 2002-060400, paragraphs 0019-0021.

Furthermore, the hybrid polypeptide is preferably a bound product of an anti-inflammatory factor or an angiogenic factor to the carboxyl terminal of FNCBD.

The anti-inflammatory factor is a cytokine with inflammation inhibitory action and may be specifically exemplified such as TGF-β, IL-4, IL-5, IL-10, IL-13, galectin-3 and N-terminal deleted chemokine.

More preferably, an N-terminal deleted chemokine inducing migration/activation of leukocyte and lymphocyte may be illustrated among these anti-inflammatory factors, furthermore preferably, 7ND that is an N-terminal deleted compound of monocyte chemoattractant protein-1 (MCP-1) may be illustrated.

The angiogenic factor is a cytokine transmitting a signal for stimulation of angiogenesis and specifically such as HGF, VEGF, bFGF, TNF-α or TP may be cited, and HGF is more preferable among them.

More preferably, the gene encoding a hybrid polypeptide is such as SEQ ID No:1 (FNCBD-7ND) or SEQ ID No:2 (FNCBD-HGF).

The stent of the present invention can be prepared by formation of a layer (a gene layer) containing the gene encoding a hybrid polypeptide on the whole surface (exterior and interior surfaces) or a partial surface (for example, exterior surface only, and can be suitably selected).

The gene layer may be one layer or combinations of plural layers composed of two or more different functions and any formation method can be used without any restriction. For example, a direct formation of the gene layer on the surface of stent using a binder component or the like, or formation of a primer layer followed by formation of the gene layer on the primer layer together with further formation of a protective layer may be applied.

The specific procedures for the formation may be suitable combinations such as spreading, dipping or spraying using water and/or an organic solvent capable of dissolution of the gene and the binder component or the like, followed by drying (natural drying or drying under reduced pressure or the like).

The stent of the present invention having a layer containing the gene encoding a hybrid polypeptide on the surface will gradually release the anti-inflammatory factor or angiogenic factor by acidosis when it is indwelled in the arterial lesion and for example, and in addition, has an enhanced efficiency of the gene transfer into cells of the lesion. Thus, the stent directly and continuously exerts anti-inflammatory action and angiogenesis action for a long period in tissues under inflammatory or hypoxic conditions. The transfer efficiency is shown by an equation of number of genes introduced in cells of the lesion/amount of released genes×100.

The stent of the present invention can be used for treatments of vascular restenosis (restenosis after percutaneous transluminal coronary angioplasty (PTCA) or percutaneous transluminal angioplasty (PTA)), acute coronary syndromes or cerebral ischemia.

The drug/gene eluting stent of the present invention has advantages that can directly deliver the genes encoding a hybrid polypeptide, particularly in uniform microcapsules of hybrid polypeptide, to the lesion and can reduce the given doses, and thus can improve safety and efficacy and further can maintain the efficacy for a long period.

### Brief Description of Drawings

Fig. 1 shows an optical microscopic photograph of an inflammatory lesion image in a metal stent group with hematoxylin and eosin stain (HE);
Fig. 2 shows an optical microscopic photograph of an inflammatory lesion image with the drug/gene eluting stent group, which is similar to Fig. 1;
Fig. 3 shows a comparison of numbers of cells positive to the monoclonal antibody against a rabbit·macrophage RAM11;
Fig. 4 shows a comparison of MCP-1 concentrations in monkey serum (pg/ml);
Fig. 5 shows an optical microscopic photograph of a blood vessel in an empty vector plasmid-coated stent group;
Fig. 6 shows a blood vessel in the drug/gene eluting stent group;
Fig. 7 shows a comparison of intimal areas in Figs. 5 and 6;
Fig. 8 shows a comparison of internal elastic lamina (IEL) areas; and
Fig. 9 shows an image for confirmation of the gene expression in the gene-coated stent.

### Examples

The present invention will be illustrated in further detail with reference to Examples below, however, the present invention is of course not limited by these Examples.

### Example 1 Preparation of a drug/gene eluting stent

A drug/gene eluting stent with four layers structure was prepared by the following steps (1) to (4);
(1) A silane coupling agent was diluted in a methanol/water (volume ratio: 50/50), applied on an exterior surface of a stent with inner diameter of about 2.8 mm, and formed a primer layer after drying.
(2) A 2 mass% solution of a water absorptive polyether type thermoplastic polyurethane dissolved in tetrahydrofuran (THF) was coated on a primer layer and formed a drug layer base.
(3) The stent prepared by step (2) in an aqueous solution of a gene encoding a hybrid polypeptide at 10 mass% was dipped and thoroughly swelled. Thereafter, the stent was vacuum dried to remove water and formed a drug layer.
(4) A 2 mass% solution of a highly in vivo stable polycarbonate type thermoplastic polyurethane dissolved in tetrahydrofuran (THF) was coated on the drug layer to form a protective layer and the stent of the present invention was obtained. The burst release of the drug can be inhibited by the protective layer.

### Example 2 Investigation of a gene expression and inflammation image in a cholesterol loaded rabbit·model

A stent (inner diameter of about 2.8 mm) coated with a gene encoded a marker gene, LacZ, was indwelled in an iliac artery of a hyperlipemic domestic rabbit loaded with 1% cholesterol for four weeks and investigated the gene expression after three days. Cells positive to gene expression were observed in 60-70% of intimal cells and 50% of medial and outer membrane cells. No positive cell was observed in a stent without gene coating. This was tenfold or over of the transfer efficiency compared to the result shown in Nat. Biotechnol., 2000, Nov.; 18(11): 1181-4.

### Example 3 Investigation of an inflammatory image in cholesterol loaded rabbit·model

Metal stents (n=5) or gene-coated stents (n=5) (both have inner diameter of about 2.8 mm) were indwelled in an iliac artery of hyperlipemic domestic rabbit loaded with 1% cholesterol for 12 weeks. Determination of macrophage infiltration and blood level of monocyte migration factor, MCP-1, were performed after 10 days.

In addition, the gene-coated stent was prepared with the metal stent similar to that of the control, and a biodegradable base material containing a plasmid composed of a gene encoding FNCBD-7ND hybrid polypeptide in a similar manner with that of the preparation example.

Macrophage infiltration in arteries was searched using an antibody which specifically recognizes rabbit macrophage in histochemical investigation and a significant inhibition of the infiltration degree (p<0.01) was observed in the gene-coated stent group in comparison with that in the metal stent group.

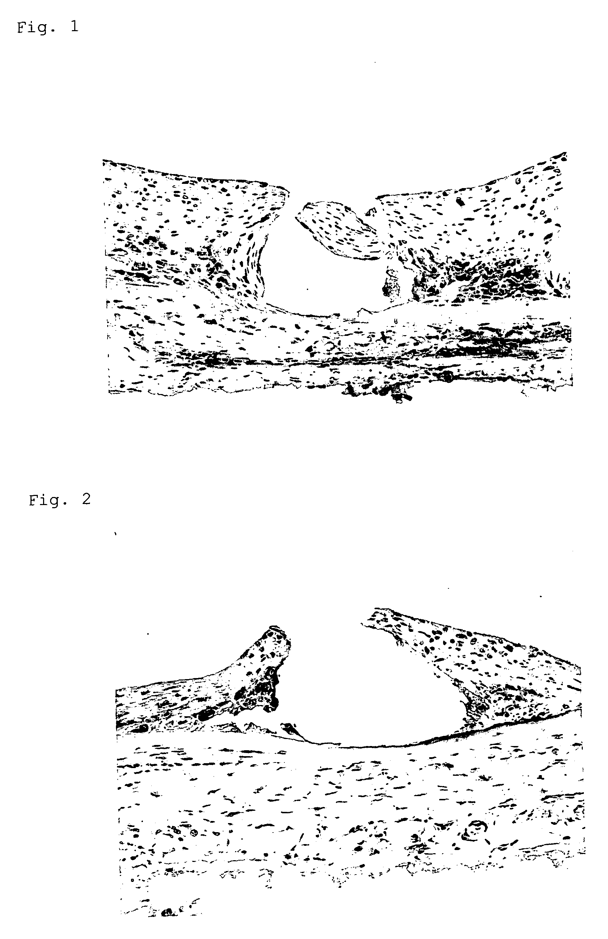

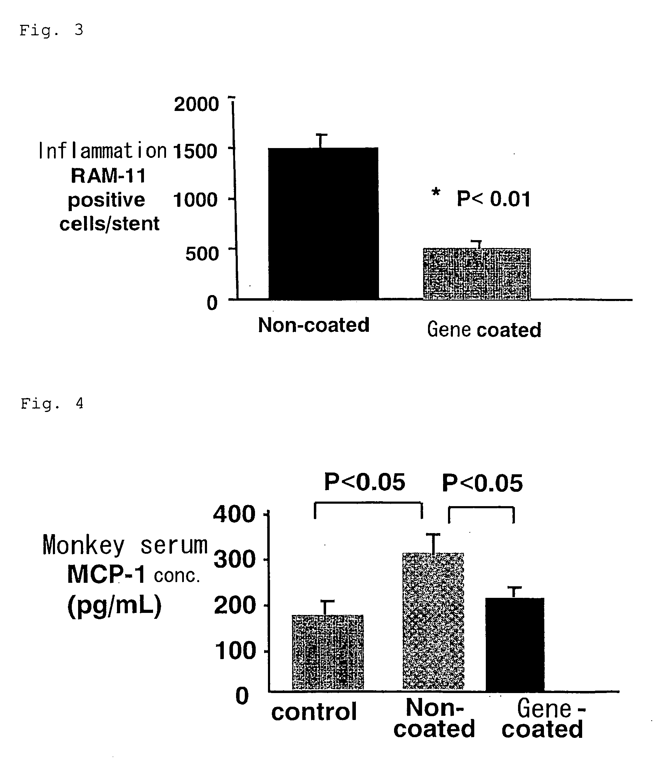

The results are shown in Figs. 1, 2, 3 or 4. Fig. 1 shows a photograph of inflammatory image in the metal stent group (in the Figure, the upper part shows lumen side and the lower part shows outer membrane side). Infiltration of the foamed macrophage under the intimal membrane is observed. Fig. 2 is a photograph showing inflammatory image in the gene-coated stent group (in the Figure, the upper part shows lumen side and the lower part shows outer membrane side). Scarcely any foamed macrophage is observed. Fig. 3 shows a comparison of the numbers of cells positive to monoclonal antibody against rabbit macrophage RAM11. Fig. 4 shows a comparison of MCP-1 concentrations (pg/ml) in monkey serum.

### Example 4 Investigation of the inflammatory images in cholesterol loaded monkey·model

Empty vector plasmid-coated stents (n=5) or gene-coated stents (similar with those in Example 3, n=5) were indwelled in an iliac artery of a crab-eating monkey *(Macaca irus)* loaded with 1% cholesterol for 12 weeks. Thickened endocardial membrane (occupiedareaofintimalmembrane) was determined after 28 days.

A significant inhibition of the thickening of endocardial membrane (p<0.01) was observed in the gene-coated stent group in comparison with that in the empty vector plasmid-coated stent group in the histochemical investigation. The blood monocyte migration factor (MCP-1) concentrations expressing inflammatory changes 10 days after the displacement of the stents were significantly inhibited in the gene-coated stent group in comparison to those in the metal stent group (p<0.05).

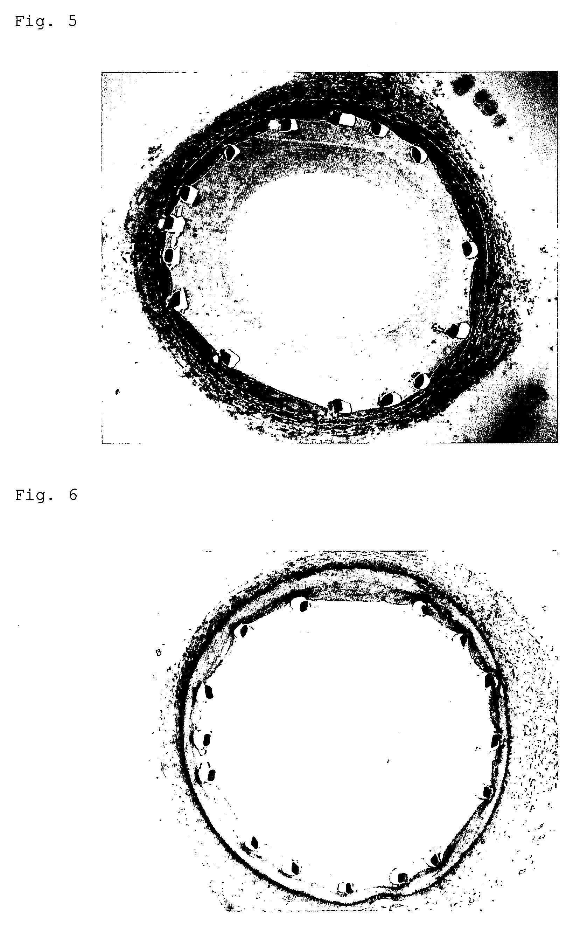

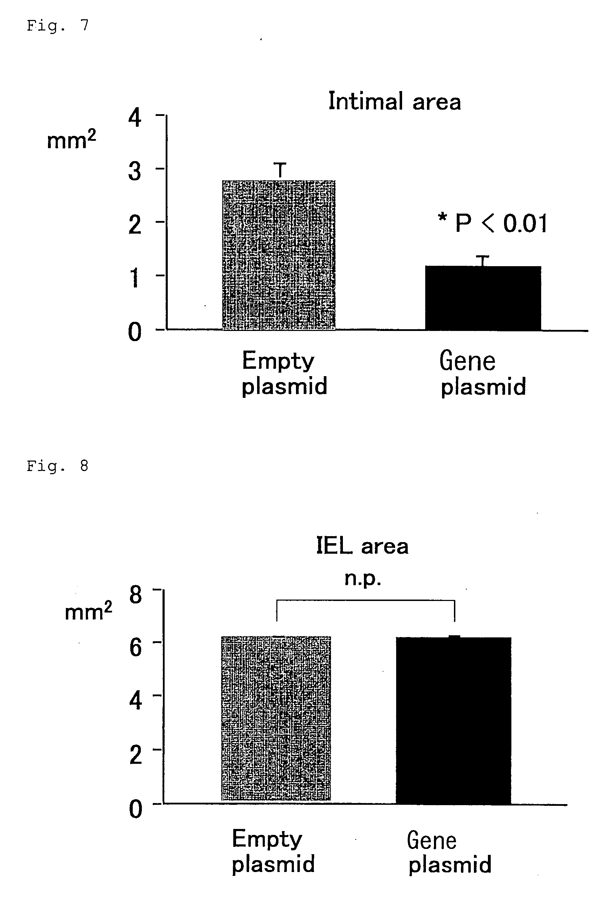

The results are shown in Figs. 5, 6, 7 or 8. Fig. 5 shows a photograph of blood vessel in the empty vector plasmid-coated stent group. Stenosis caused by the thickened intimal membrane was observed. Fig. 6 shows a blood vessel of the gene-coated stent group. No stenosis was observed. Fig. 7 shows a comparison of intimal membrane areas between those in Fig. 5 and 6 (average ± standard deviation, the empty vector plasmid-coated stent group: 2.79±0.92, the gene-coated stent group: 1.59±0.77). Fig. 8 shows comparison of areas of internal elastic lamina: IEL).

### Comparative Experimental Example 1

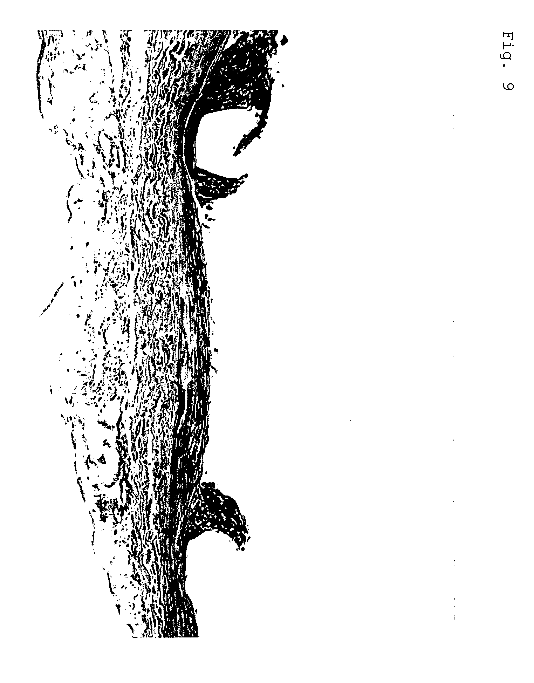

A stent coated with FNCBD-GFP plasmid was prepared according to Example 1 and the transfer efficiency was evaluated by β-gal staining in an enlarged cross-sectional image according to Experimental protocol in Nat. Biotechnol., 2000 Nov.; 18(11): 1181-4 (Fig. 9). A stent without gene coating was used as a control.

As a result, 62±12% in intimal membrane cells (n=3) and 54±9% in medial and outer membrane cells (n=3) in the FNCBD-GFP plasmid-coated stent group were GFP positive, however, no positive cell was observed in stents without gene coating (0%). On the other hand, the gene transfer efficiency of the GFP plasmid-coated stent is 7.9±0.7% according to Nat. Biotechnol. , 2000 Nov.; 18(11): 1181-4.

The present invention makes up a high performance drug delivery system (DDS) with substantial nanocapsules using a gene and different from conventional drug-coated stents.

## Claims

1. A drug/gene eluting stent comprising a layer containing a gene encoding a hybrid polypeptide on the surface.

2. The drug/gene eluting stent according to claim 1, wherein the hybrid polypeptide is a binding of a fibronectin-derived collagen binding domain (FNCBD) polypeptide and an anti-inflammatory factor or an angiogenic factor.

3. The drug/gene eluting stent according to claim 1 or 2, wherein the hybrid polypeptide is a bound product of an anti-inflammatory factor or an angiogenic factor to a carboxyl terminal of FNCBD.

4. The drug/gene eluting stent according to claim 2 or 3, wherein the anti-inflammatory factor is a N-terminal deleted chemokine.

5. The drug/gene eluting stent according to claim 4, wherein the N-terminal deleted chemokine is N-terminal deleted compound (7ND) of a monocyte chemoattractant protein-1 (MCP-1).

6. The drug/gene eluting stent according to any one of claims 1 to 5, wherein the gene encoding the hybrid polypeptide has the sequence showin in SEQ ID No: 1 or 2.

7. The drug/gene eluting stent according to any one of claims 1 to 6, **characterized by** being used for treatment of vascular restenosis, acute coronary syndromes or cerebral ischemia.

8. The drug/gene eluting stent according to claim 7, wherein the vascular restenosis is a relapsed stenosis of post percutaneous transluminal coronary angioplasty (PTCA) or percutaneous transluminal angioplasty (PTA).

9. A method for treating vascular restenosis, acute coronary syndromes or cerebral ischemia, which comprises using the drug/gene eluting stent according to any one of claims 1 to 6.

10. Use of the drug/gene eluting stent according to any one of claims 1 to 6 for manufacturing an agent for treating vascular restenosis, acute coronary syndromes or cerebral ischemia.


SEQUENCE LISTING

<110> Kensuke Egashira

<120> Drug-gene eluting stent

<130> 04108PCT

<150> JP 2004-077581
<151> 2004-3-18

<160> 2

<210> 1
<211> 1254
<212> DNA
<213> Homo Sapiens
<223> FNCBD-7ND

<400> 1
atggcagctg tttaccaacc gcagcctcac ccccagcctc ctccctatgg ccactgtgtc 60
acagacagtg gtgtggtcta ctctgtgggg atgcagtggc tgaagacaca aggaaataag 120
caaatgcttt gcacgtgcct gggcaacgga gtcagctgcc aagagacagc tgtaacccag 180
acttacggtg gcaactcaaa tggagagcca tgtgtcttac cattcaccta caatggcagg 240
acgttctact cctgcaccac ggaagggcga caggacggac atctttggtg cagcacaact 300
tcgaattatg agcaggacca gaaatactct ttctgcacag accacactgt tttggttcag 360
actcgaggag gaaattccaa tggtgccttg tgccacttcc ccttcctata caacaaccac 420
aattacactg attgcacttc tgagggcaga agagacaaca tgaagtggtg tgggaccaca 480
cagaactatg atgccgacca gaagtttggg ttctgcccca tggctgccca cgaggaaatc 540
tgcacaacca atgaaggggt catgtaccgc attggagatc agtgggataa gcagcatgac 600
atgggtcaca tgatgaggtg cacgtgtgtt gggaatggtc gtggggaatg gacatgcatt 660
gcctactcgc agcttcgaga tcagtgcatt gttgatgaca tcacttacaa tgtgaacgac 720
acattccaca agcgtcatga agaggggcac atgctgaact gtacatgctt cggtcagggt 780
cggggcaggt ggaagtgtga tcccgtcgac caatgccagg attcagagac tgggacgttt 840
tatcaaattg gagattcatg ggagaagtat gtgcatggtg tcagatacca gtgctactgc 900
tatggccgtg gcattgggga gtggcattgc caacctttac agacctatcc aagctcaagt 960
ggtcctgtcg aagtatttat cactgagact ccgagtcagc ccaactccca ccccatccag 1020
tggggatccg acgacgatga taagcaggtc acctgctgtt ataacttcac caataggaag 1080
atctcagtgc agaggctcgc gagctataga agaatcacca gcagcaagtg tcccaaagaa 1140
gctgtgatct tcaagaccat tgtggccaag gagatctgtg ctgaccccaa gcagaagtgg 1200
gttcaggatt ccatggacca cctggacaag caaacccaaa ctccgaagac ttga 1254

<210> 2
<211> 3138
<212> DNA
<213> Homo Sapiens
<223> FNCBD-HGF

<400> 2
atggcagctg tttaccaacc gcagcctcac ccccagcctc ctccctatgg ccactgtgtc 60
acagacagtg gtgtggtcta ctctgtgggg atgcagtggc tgaagacaca aggaaataag 120
caaatgcttt gcacgtgcct gggcaacgga gtcagctgcc aagagacagc tgtaacccag 180
acttacggtg gcaactcaaa tggagagcca tgtgtcttac cattcaccta caatggcagg 240
acgttctact cctgcaccac ggaagggcga caggacggac atctttggtg cagcacaact 300
tcgaattatg agcaggacca gaaatactct ttctgcacag accacactgt tttggttcag 360
actcgaggag gaaattccaa tggtgccttg tgccacttcc ccttcctata caacaaccac 420
aattacactg attgcacttc tgagggcaga agagacaaca tgaagtggtg tgggaccaca 480
cagaactatg atgccgacca gaagtttggg ttctgcccca tggctgccca cgaggaaatc 540
tgcacaacca atgaaggggt catgtaccgc attggagatc agtgggataa gcagcatgac 600
atgggtcaca tgatgaggtg cacgtgtgtt gggaatggtc gtggggaatg gacatgcatt 660
gcctactcgc agcttcgaga tcagtgcatt gttgatgaca tcacttacaa tgtgaacgac 720
acattccaca agcgtcatga agaggggcac atgctgaact gtacatgctt cggtcagggt 780

cggggcaggt ggaagtgtga tcccgtcgac caatgccagg attcagagac tgggacgttt 840
tatcaaattg gagattcatg ggagaagtat gtgcatggtg tcagatacca gtgctactgc 900
tatggccgtg gcattgggga gtggcattgc caacctttac agacctatcc aagctcaagt 960
ggtcctgtcg aagtatttat cactgagact ccgagtcagc ccaactccca ccccatccag 1020
tggggatccg acgacgatga taagcaaagg aaaagaagaa atacaattca tgaattcaaa 1080
aaatcagcaa agactaccct aatcaaaata gatccagcac tgaagataaa aaccaaaaaa 1140
gtgaatactg cagaccaatg tgctaataga tgtactagga ataaaggact tccattcact 1200
tgcaaggctt ttgtttttga taaagcaaga aaacaatgcc tctggttccc cttcaatagc 1260
atgtcaagtg gagtgaaaaa agaatttggc catgaatttg acctctatga aaacaaagac 1320
tacattagaa actgcatcat tggtaaagga cgcagctaca agggaacagt atctatcact 1380
aagagtggca tcaaatgtca gccctggagt tccatgatac cacacgaaca cagctttttg 1440
ccttcgagct atcggggtaa agacctacag gaaaactact gtcgaaatcc tcgaggggaa 1500
gaagggggac cctggtgttt cacaagcaat ccagaggtac gctacgaagt ctgtgacatt 1560
cctcagtgtt cagaagttga atgcatgacc tgcaatgggg agagttatcg aggtctcatg 1620
gatcatacag aatcaggcaa gatttgtcag cgctgggatc atcagacacc acaccggcac 1680
aaattcttgc ctgaaagata tcccgacaag ggctttgatg ataattattg ccgcaatccc 1740
gatggccagc cgaggccatg gtgctatact cttgaccctc acacccgctg ggagtactgt 1800
gcaattaaaa catgcgctga caatactatg aatgacactg atgttccttt ggaaacaact 1860
gaatgcatcc aaggtcaagg agaaggctac aggggcactg tcaataccat ttggaatgga 1920
attccatgtc agcgttggga ttctcagtat cctcacgagc atgacatgac tcctgaaaat 1980
ttcaagtgca aggacctacg agaaaattac tgccgaaatc cagatgggtc tgaatcaccc 2040
tggtgtttta ccactgatcc aaacatccga gttggctact gctcccaaat tccaaactgt 2100
gatatgtcac atggacaaga ttgttatcgt gggaatggca aaaattatat gggcaactta 2160
tcccaaacaa gatctggact aacatgttca atgtgggaca agaacatgga agacttacat 2220
cgtcatatct tctgggaacc agatgcaagt aagctgaatg agaattactg ccgaaatcca 2280
gatgatgatg ctcatggacc ctggtgctac acgggaaatc cactcattcc ttgggattat 2340
tgccctattt ctcgttgtga aggtgatacc acacctacaa tagtcaattt agaccatccc 2400
gtaatatctt gtgccaaaac gaaacaattg cgagttgtaa atgggattcc aacacgaaca 2460
aacataggat ggatggttag tttgagatac agaaataaac atatctgcgg aggatcattg 2520
ataaaggaga gttgggttct tactgcacga cagtgtttcc cttctcgaga cttgaaagat 2580
tatgaagctt ggcttggaat tcatgatgtc cacggaagag gagatgagaa atgcaaacag 2640
gttctcaatg tttcccagct ggtatatggc cctgaaggat cagatctggt tttaatgaag 2700
cttgccaggc ctgctgtcct ggatgatttt gttagtacga ttgatttacc taattatgga 2760
tgcacaattc ctgaaaagac cagttgcagt gtttatggct ggggctacac tggattgatc 2820
aactatgatg gcctattacg agtggcacat ctctatataa tgggaaatga gaaatgcagc 2880
cagcatcatc gagggaaggt gactctgaat gagtctgaaa tatgtgctgg ggctgaaaag 2940
attggatcag gaccatgtga gggggattat ggtggcccac ttgtttgtga gcaacataaa 3000
atgagaatgg ttcttggtgt cattgttcct ggtcgtggat gtgccattcc aaatcgtcct 3060
ggtatttttg tccgagtagc atattatgca aaatggatac acaaaattat tttaacatat 3120
aaggtaccac agtcatag 3138